# EUROPÄISCHE PATENTSCHRIFT

(11) **EP 3 747 454 B1**
(45) Veröffentlichungstag und Bekanntmachung des Hinweises auf die Patenterteilung: **07.06.2023**
(21) Anmeldenummer: 20180219.6
(22) Anmeldetag: 23.09.2014
(51) Int. Cl.: A61K 38/10, A61K 38/16, A61K 9/20, A61K 9/00, A61K 9/10, A61P 25/28, A61P 31/18, C07K 7/08, C07K 7/64, C07K 14/00, A61K 38/12, G01N 33/68

(54) **AMYLOID-BETA-BINDENDE PEPTIDE UND DIESE PEPTIDE ZUR VERWENDUNG FÜR DIE THERAPIE UND DIE DIAGNOSE DER ALZHEIMERSCHEN DEMENZ**
AMYLOID-BETA BINDING PEPTIDES AND THESE PEPTIDES FOR USE IN THE THERAPY AND DIAGNOSIS OF ALZHEIMER'S DEMENTIA
PEPTIDES LIANTS AMYLOÏDES-BÊTA ET CES PEPTIDES POUR LEUR UTILISATION POUR LE TRAITEMENT ET LE DIAGNOSTIC DE LA MALADIE D'ALZHEIMER

(30) Priorität: 26.09.2013 DE 102013016002; 12.03.2014 DE 102014003262
(43) Veröffentlichungstag der Anmeldung: 09.12.2020
(62) Teilanmeldung aus: 14799955.1
(73) Patentinhaber: Priavoid GmbH, 40225 Düsseldorf (DE)
(72) Erfinder: WILLBOLD, Dieter, 52428 Jülich (DE)
(74) Vertreter: Fitzner, Uwe

(56) Entgegenhaltungen:
- WO-A2-2005/060683
- WO-A2-2006/087550
- WO-A2-2013/150126
- WO-A2-2013/150127

## Beschreibung

Die Erfindung bezieht sich auf Amyloid-Beta-bindende Peptide und deren Verwendung für die Therapie und die Diagnose der Alzheimerschen Demenz.

### Stand der Technik

Aus der Offenlegungsschrift WO 2013/150127 A2 sind multivalente Amyloid-Beta-bindende polymere Substanzen, bestehend aus mehreren miteinander verbundenen Substanzen, die für sich bereits Amyloid-Beta-bindende Eigenschaften haben, sowie die Verwendung dieser Substanzen insbesondere in der Medizin, bekannt.

Aufgrund der demographischen Entwicklung in den nächsten Jahrzehnten wird sich die Zahl der Personen, die an altersbedingten Erkrankungen leiden, erhöhen. Hier ist insbesondere die sog. Alzheimer-Krankheit (AD, Alzheimersche Demenz, lateinisch = Morbus Alzheimer) zu nennen.

Bisher existiert kein Wirkstoff oder Medikament, das gegen die Ursachen von AD wirkt. Die bisher eingesetzten und zugelassenen Medikamente mildern einige der bei der Alzheimerschen Demenz auftretenden Symptome. Sie sind aber nicht in der Lage, den Krankheitsfortschritt zu verlangsamen oder eine Heilung herbeizuführen. Es existieren einige Substanzen, die im Tierversuch Erfolge bei der Prävention, aber nicht (unbedingt) bei der Behandlung von AD gezeigt haben. Wirkstoffe gegen neurodegenerative Erkrankungen sind aus der DE 10 2006 015 140 A1 bekannt.

Ein Merkmal der Alzheimer-Erkrankung sind extrazelluläre Ablagerungen des Amyloid-Beta-Peptids (A-Beta-Peptid, Aß oder Aß-Peptid). Diese Ablagerung des A-Beta-Peptids in Plaques ist typischerweise in den Gehirnen von AD-Patienten *post mortem* festzustellen. Deshalb werden verschiedene Formen des A-Beta-Peptids - wie z.B. Fibrillen - für die Entstehung und das Fortschreiten der Krankheiten verantwortlich gemacht. Zusätzlich werden seit einigen Jahren die kleinen, frei diffundierbaren A-Beta-Oligomere als hauptsächliche Verursacher der Entstehung und des Fortschritts der AD gesehen.

Die aus dem Stand der Technik bekannten Substanzen verringern die Konzentration von ABeta-Monomeren und/oder Oligomeren auf verschiedenste Art und Weise. So sind z.B. Gamma-Sekretase-Modulatoren bekannt, die im Tierversuch zur Prävention eingesetzt wurden.

Aus der WO 02/081505 A2 sind verschiedene Sequenzen von D-Aminosäuren bekannt, die an A-Beta-Peptide binden. Diese Sequenzen aus D-Aminosäuren binden mit einer Dissoziationskonstante (K_{D}-Wert) von 4 µM an Amyloid-Beta-Peptide.

Aus der WO 2011/147797 A2 sind Hybridverbindungen bekannt, bestehend aus Aminopyrazolen und Peptiden, die A-Beta-Oligomerisation verhindern.

Verbindungen, die mit A-Beta-Peptiden interagieren, sind aus der DE 10 2008 037 564 A1, DE 696 21 607 T2 oder DE 10 2010 019 336 A1 bekannt. Die Bindung eines multivalenten Polymers an zwei Bindungspartner wird in der WO 2008/116293 A1 beschrieben.

Bei vielen Substanzen, die im Tierversuch positive Ergebnisse gezeigt haben, konnte diese Wirkung in klinischen Studien am Menschen nicht bestätigt werden. In klinischen Studien der Phase II und III dürfen nur Menschen behandelt werden, die eindeutig mit AD diagnostiziert wurden. Hier reicht eine geringe Verringerung der A-Beta-Monomerkonzentration nicht mehr aus, um zu verhindern, dass sich aus den bereits vorhandenen A-Beta-Oligomeren noch mehr bilden, z.B. durch einen Prion-ähnlichen Mechanismus. Die Vermehrung der A-Beta-Oligomere oder noch besser ihre Zerstörung oder Unschädlichmachung ist aber unbedingt erforderlich, um Einfluss auf den Krankheitsverlauf zu nehmen.

Bisher wird die Alzheimersche Demenz hauptsächlich durch neuro-psychologische Tests diagnostiziert, durch Experimente an Personen, bei denen Demenzsymptome erkannt wurden. Es ist jedoch bekannt, dass A-Beta-Oligomere und die zeitlich darauf folgenden Fibrillen und Plaques bis zu 20 Jahre vor dem Auftreten der Symptome im Gehirn der Patienten entstehen, und schon irreversible Schäden verursacht haben können. Allerdings gibt es bisher noch keine Möglichkeit, die AD vor dem Ausbruch der Symptome zu diagnostizieren.

Es besteht somit weiterhin ein Bedarf an neuen Verbindungen (Wirkstoffen), die sehr spezifisch und mit hoher Affinität an A-Beta-Oligomere binden, und so deren Vermehrung verhindern. Diese Verbindungen sollten keine Effekte mit unerwünschten Nebenwirkungen zeigen, insbesondere keine Immunreaktion hervorrufen. Die Verbindungen sollen außerdem toxische A-Beta-Oligomere und damit auch die kleinen frei diffundierbaren Oligomere auch in kleinen Konzentrationen erkennen, vollständig vernichten und/oder deren (Prionen-ähnliche) Vermehrung verhindern.

Des Weiteren besteht auch ein Bedarf an neuen Verbindungen, die als Sonden für die Erkennung und Markierung von A-Beta-Oligomeren einsetzbar sind, insbesondere, wenn diese Oligomere erst in kleinen Konzentrationen auftreten.

WO 2013/021353 A1 betrifft oberflächenmodifizierte proteinöse spherische Partikel, die eine Proteinschicht und eine amyloidbindende Gruppe, die an das Protein gebunden ist, umfassen können.

WO 01/34631 A2 offenbart ein inhibitorisches Peptid, das fähig ist, beta-Faltbblattbildung in Amyloid-beta-Peptid zu inhibieren, ein inhibitorisches Peptid, das in der Lage ist, Änderungen der Konformation in Prionprotein PrP, das mit Amyloidose assoziiert ist, zu inhibieren, sowie Peptidmimetika.

Kapurniotu A. et al. (Conformational Restriction via Cyclization in sz-Amyloid Peptide Asz(1-28) Leads to an Inhibitor of Asz(1-28) Amyloidogenesis and Cytotoxicity, Chemistry and Biology, Current Biology, Bd. 10, Nr. 2, 1. Februar 2003, Seiten 149 bis 159) offenbaren ein zyklisches beta-Amyloid-Peptid, das an Abeta1-40 bindet und Amyloidaggregation verhindert, für Therapie und Diagnostik.

Rahimipour S. et al. (In Vitro and Mechanistic Studies of an Anti-Amyloidogenic Self-Assembled Cyclic D,L-alpha-Peptide Architecture, Biopolymers, Bd. 100, Nr. 3, 1. Mai 2013, Seite 230) beschreiben ein spezifisches zyklisches D,L-alpha-Peptid, das an Abeta bindet.

WO 02081505 A2 betrifft Peptide, die das Amyloid-beta-Peptid mit hoher Affinität binden, ein Verfahren zur Herstellung solcher Peptide und die Verwendung solcher Peptide für die Diagnostik und Therapie von Morbus Alzheimer.

Sievers Stuart A. et al (Structure-based design of non-natural amino-acid inhibitors of amyloid fibril formation, Nature, Bd. 475, Nr. 7354, 1. Juli 2011, Seiten 96 bis 100) beschreiben Inhibitoren der Amyloid-Fibrillen-Bildung.

WO 00/36093 A2 offenbart Verfahren zur Herstellung zyklischer Peptide.

DE 10 2012 102998 A1 offenbart ein Polymer, das mindestens zwei Monomere umfasst, die an Amyloid-beta-Oligomere binden.

DE 10 2012 102999 A1 betrifft ein Verfahren zur Behandlung (ex vivo) von Blut, Blutprodukten und Organen zur Prävention der Übertragung von Morbus-Alzheimer und anderen Amyloid-basierten Erkrankungen.

WO 2006/087550 A2 betrifft chemische Verbindungen und Zusammensetzungen, umfassend amyloidbindende Peptidsequenzen oder Analoga und Derivative davon.

WO 2007/145589 A1 betrifft neue Peptide, die an ein Amyloid-beta-Peptid binden können, wobei das Na-Atom mindestens eines Aminosäurerests durch einen Substituenten substituiert ist, der die weitere Bindung des Amyloid-beta-Peptids sterisch hindert.

WO 2014/177127 A1 betrifft ein Mittel zur Prophylaxe und/oder Behandlung von HIV und anderen Virusinfektionen. Das Mittel enthält insbesondere mindestens ein Peptid mit einer Aminosäuresequenz, die zur Prophylaxe von mit Alzheimer-Krankheit assoziierten Fibrillen geeignet ist, und/oder Homologe, Fragmente und Teile dieses Peptids.

WO 2013/150126 A2 betrifft die Behandlung von Blut, Blutprodukten und Organen zur Entfernung und/oder Entgiftung von Amyloid-beta-Oligomeren.

WO 2005/060683 A2 betrifft die Verwendung von 5- bis 13-meren Peptiden und Peptidderivaten zur Behandlung der Alzheimerkrankheit.

### Aufgabe der Erfindung

Aufgabe der Erfindung ist es daher Verbindungen anzugeben für
A) Die ursächliche Therapie von Morbus Alzheimer, indem diese Verbindungen die Bildung von toxischen Amyloid-Beta-Oligomeren, Fibrillen oder Aggregaten verhindern oder bereits entstandene Oligomere, Fibrillen oder Aggregate eliminieren oder deren Enttoxifizierung bewirken.
B) Die Diagnose der Alzheimerschen Demenz erlauben, in dem diese Verbindungen als Sonde für in vivo Imaging eingesetzt werden können.

Hiermit sind weitere Aufgaben, wie die Verwendung der Verbindungen in der Medizin umfasst.

Aufgabe der Erfindung ist es außerdem neue Peptide zur Verfügung zu stellen, bevorzugt Derivate des D-enantiomeren D-Peptids D3, die im Vergleich zu D3 effizientere Eigenschaften besitzen. Die Eigenschaften umfassen unter anderem höhere Bindungsaffinität und - spezifität für A-Beta-Spezies, Inhibition der A-Beta-Fibrillenbildung, Inhibierung der A-Beta-Zytotoxizität, Eliminierung oder Enttoxifizierung von A-Beta-Oligomeren, Fibrillen und anderen Aggregaten, Umwandlung von A-Beta-Amyloid-Fibrillen, -Protofibrillen, oder -Oligomere in nicht-toxische, nicht-amyloidogene Spezies.

Im Nachfolgenden werden die Begriffe "A-Beta", "Amyloid-Beta", "Amyloid-β" bzw. "Aβ" synonym zueinander gebraucht.

### Lösung der Aufgabe

Die Aufgabe wird gelöst mit dem Peptid, dem Kit und der Zusammensetzung gemäß der Hauptansprüche sowie den Verfahren und Verwendungen gemäß den Nebenansprüchen. Vorteilhafte Ausgestaltungen hierzu ergeben sich jeweils aus den hierauf rückbezogenen Patentansprüchen.

### Beschreibung der Erfindung

### [1] Das erfindungsgemäße Peptid ist ein

Peptid, enthaltend mindestens eine Aminosäuresequenz, welche an Amyloid-Beta-Spezies bindet und bei dem die negative Ladung der gewöhnlich am freien C-Terminus vorhandenen Carboxylgruppe durch Modifizierung entfernt wurde, so dass dort keine oder eine positive Ladung vorkommt, wobei am freien C-terminus an Stelle der Carboxylgruppe eine Säureamidgruppe (CONH2-Gruppe) vorliegt,
enthaltend mindestens ein Monomer mit einer Aminosäuresequenz, ausgewählt aus der Gruppe bestehend aus SEQ ID NO: 4, SEQ ID NO: 5 und SEQ ID NO: 6.

Ein Peptid gemäß der Offenbarung, ist ein Peptid, enthaltend mindestens eine Aminosäuresequenz, welche an Amyloid-Beta-Spezies bindet, und diese Eigenschaft entweder erhalten bleibt oder verstärkt wird, und bei der der freie C-terminus, also die C-terminale Carboxylguppe, derart modifiziert ist, dass der C-terminus keine negative Ladung trägt, sondern stattdessen neutral ist oder eine oder mehrere positive Ladungen aufweist.

Unter die erfindungsgemäßen Peptide fallen auch solche Peptide, umfassend mindestens ein an eine Amyloid-Beta-Spezies bindendes Peptid, wobei das Peptid eine lineare Aminosäuresequenz aufweist, die es dazu befähigt, an A-Beta zu binden, und diese Eigenschaft entweder erhalten bleibt oder verstärkt wird, in dem das Peptid durch kovalente Verknüpfung seiner beiden Enden in zyklisierter Form vorliegt.

Durch die erfindungsgemäßen Peptide wird besonders vorteilhaft zusätzlich die Aufgabe gelöst, dass ein Peptid ohne negative Ladung am C-terminus, bereitgestellt wird. Dadurch wird vorteilhaft bewirkt, dass dieses mit höherer Affinität an das Zielmolekül binden kann, als ein Peptid, welches eine Carboxylgruppe am freien C-terminus aufweist. Peptide mit freier, nicht-modifizierter Carboxylgruppe weisen im physiologischen Zustand eine negative Ladung an diesem Ende auf.

In einer Ausgestaltung der Erfindung sind die erfindungsgemäßen Peptide im physiologischen Zustand, insbesondere bei pH 6-8, insbesondere 6,5-7,5, insbesondere bei pH 6,0, pH 6,1, pH 6,2, pH 6,3, pH 6,4, pH 6,5 pH 6,6, pH 6,7, pH 6,8, pH 6,9, pH 7,0, pH 7,1, pH 7,2, pH 7,3, pH 7,4, pH 7,5, pH 7,6, pH 7,7, pH 7,8, pH 7,9 bzw. pH 8,0 derart modifiziert, dass der C-terminus keine negative Ladung trägt, sondern stattdessen neutral ist oder eine oder mehrere positive Ladungen aufweist.

Erfindungsgemäß ist das Peptid dadurch gekennzeichnet, dass am freien C-terminus an Stelle der Carboxylgruppe eine Säureamidgruppe vorliegt. Statt der Carboxylgruppe (-COOH-Gruppe) ist also eine Säureamidgruppe (-CONH2-Gruppe) am C-terminus angeordnet.

Das Peptid ist demnach besonders vorteilhaft am freien C-Terminus amidiert.

Dadurch wird besonders vorteilhaft die weitere Aufgabe gelöst, dass ein Peptid ohne negativen Ladungsüberschuss vorliegt, welches affiner an das Zielmolekül binden kann und auf einfache Weise erhältlich ist.

In einer weiteren Ausgestaltung der Offenbarung (nicht erfindungsgemäß) liegen folgende weitere Gruppen an Stelle der Carboxylgruppe vor: COH, COCI, COBr, CONH-alkyl-Rest, CONH-alkyl-Amin-Rest (positive Netto-Ladung) etc, wobei man hierauf nicht beschränkt, sofern der technischen Lehre des Hauptanspruchs gefolgt wird.

Offenbart wird insbesondere ein Peptid (nicht erfindungsgemäß) enthaltend eine Aminosäuresequenz gemäß SEQ ID NO: 1 (RD2), SEQ ID NO: 2 (D3), SEQ ID NO: 3 (DB3), SEQ ID NO: 9 (D3r), SEQ ID NO: 10 (D3p), SEQ ID NO: 11 (D3a), und / oder SEQ ID NO: 12 (D3p2k) und/oder Homologe, Fragmente und Teile davon. Diese Peptide (nicht erfindungsgemäß)sind Substanz-Einheiten (im Folgenden oft "Monomere" genannt), die an Amyloid-Beta-Spezies binden.

In einer Variante der Erfindung werden solche Monomere eingesetzt, die an ein A-Beta-Monomer und/oder A-Beta-Oligomere und/oder Fibrillen des A-Beta-Peptids mit einer Dissoziationskonstante (Ko-Wert) von höchstens 500 µM, bevorzugt 250, 100, 50 µM, besonders bevorzugt 25, 10, 6 µM, insbesondere 4, 2, 1 µM oder sub-µM bindet.

Gelöst wird die Aufgabe insbesondere auch durch Polymere, die aus zwei oder mehreren der oben genannten Monomere bzw. erfindungsgemäßen Peptide bestehen, insbesondere durch ein erfindungsgemäßes Dimer der SEQ ID NO: 4 (RD2D3), SEQ ID NO: 5 (D3RD2) und/oderSEQ ID NO: 6 (D3D3). Die Dimere sind aus zwei Monomer-Einheiten aufgebaut, die jeweils an Amyloid-Beta-Spezies binden.

Die erfindungsgemäß aufgebauten Polymere aus Monomeren, die ihrerseits an A-Beta-Oligomere binden, zeigen deutliche, synergetische Effekte in Bezug auf ihre Selektivität und Affinität zu den A-Beta-Oligomeren im Vergleich zu den Monomeren. Mit anderen Worten: Die Polymere, insbesondere die Dimere ausgewählt aus der Gruppe von SEQ ID NO:4, SEQ ID NO: 5 und/oder SEQ ID NO: 6sind den Monomeren, aus denen sie aufgebaut sind, überlegen. Synergetische Effekte im Sinne der vorliegenden Erfindung sind Effekte, die eine höhere Selektivität und / oder Affinität bezüglich relevanter A-Beta-Spezies zeigen, insbesondere des Ko-Wertes betreffend die Bindung an A-Beta-Spezies im Vergleich zu den einzelnen Monomer-Einheiten.

In einer weiteren besonders vorteilhaften Ausgestaltung der Erfindung wirken die Polymere und insbesondere Dimere im Tiermodellversuch (*in vitro* bzw. *in vivo*) vorteilhaft effizienter als die Monomere.

In einer Variante der Erfindung werden solche Polymere eingesetzt, die an ein A-Beta-Monomer und/oder A-Beta-Oligomere und/oder Fibrillen des A-Beta-Peptids mit einer Dissoziationskonstante (Ko-Wert) von höchstens 500 µM, bevorzugt 250, 100, 50 µM, besonders bevorzugt 25, 10, 1 µM, besonders bevorzugt mit einer Dissoziationskonstante (K_{D}-Wert) von höchstens 500 nM, 250, 100, 50, besonders bevorzugt 25, 10, 1 nM, 500pM, 100, 50, 25, 20, 15, 10, 9, 8, 7, 6, 5, 4, 3, 2, 1 pM bis sub-pM bindet, wobei jeder Zwischenwert angenommen werden kann.

In einer Ausgestaltung der Erfindung ist die Affinität über die Dissoziationskonstante (K_{D}-Wert) definiert.

Die Dissoziationskonstante (K_{D}-Wert) des erfindungsgemäßen Peptids ist dabei in einer vorteilhaften Ausgestaltung der Erfindung im Vergleich zu linearen, bindenden Peptiden bei denen der freie C-terminus, also die C-terminale Carboxylguppe eine negative Ladung aufweist, vorteilhaft erniedrigt. Damit verbunden sind verbesserte Eigenschaften der erfindungsgemä-ßen Peptide, wie höhere Affinität der Bindung und höhere Effektivität des Abbaus und / oder der Verhinderung der Bildung toxischer Amyloid-Beta-Spezies. Dies betrifft insbesondere aber nicht ausschließlich einen niedrigeren K_{D}-Wert an der high affinity site des A-Beta (Monomer, Oligomer und Fibrillen).

Fragmente und Teile zeigen vorteilhaft eine ähnliche beziehungsweise identische Wirkung wie die erfindungsgemäßen Peptide.

In einer Variante der Offenbarung bestehen die erfindungsgemäßen und die nichterfindungsgemäßen Peptide, insbesondere die Peptide gemäß der SEQ ID NO: 1 (nicht erfindungsgemäß), SEQ ID NO: 2 (nicht erfindungsgemäß), SEQ ID NO: 3 (nicht erfindungsgemäß), SEQ ID NO: 4, SEQ ID NO: 5, SEQ ID NO: 6, SEQ ID NO: 7 (nicht erfindungsgemäß), SEQ ID NO: 8 (nicht erfindungsgemäß), SEQ ID NO: 9 (nicht erfindungsgemäß), SEQ ID NO: 10 (nicht erfindungsgemäß), SEQ ID NO: 11 (nicht erfindungsgemäß)und / oder SEQ ID NO: 12 (nicht erfindungsgemäß) und deren Homologe (nicht erfindungsgemäß), Fragmente (nicht erfindungsgemäß) oder Teile davon (nicht erfindungsgemäß) im Wesentlichen, bevorzugt mindestens 50%, 60%, 75%, 80%, besonders bevorzugt 85%, 90%, 95%, insbesondere 96%, 97%, 98%, 99%, 100% aus D-enantiomeren Aminosäuren.

Ein Polymer im Sinne der Erfindung ist gebildet aus 2, 3, 4, 5, 6, 7, 8, 9, 10, 11, 12, 13, 14, 15, 16, 17, 18, 19, 20 oder mehr Monomeren, die an sich an Amyloid-Beta-Spezies, insbesondere Oligomer binden.

Das Polymer ist insbesondere aus Monomeren gebildet ausgewählt aus der Gruppe bestehend aus SEQ ID NO: 1, SEQ ID NO: 2, SEQ ID NO: 3, SEQ ID NO: 9, SEQ ID NO: 10, SEQ ID NO: 11 und /oder SEQ ID NO: 12.

Das Polymer kann in einer Ausgestaltung der Offenbarung ausgewählt sein aus der Gruppe der SEQ ID NO: 4, SEQ ID NO: 5 und/oder SEQ ID NO: 6.

Die erfindungsgemäßen Peptide gemäß der SEQ ID NO: 4, 5 und 6, sowie die nicht erfindungsgemäßen Peptide der SEQ ID: 1, 2, 3, 7, 8, 9, 10, 11 und 12 sind in einer Ausgestaltung aus D-enantiomeren Aminosäuren aufgebaut.

Die erfindungsgemäßen Peptide gemäß der SEQ ID NO: 4, 5 und 6, sowie die nicht erfindungsgemäßen Peptide der SEQ ID: 1, 2, 3, 7, 8, 9, 10, 11 und 12sind am freien C-terminus mit einer Säureamidgruppe versehen. Monomere, beispielweise D3, DB3 oder RD2 gemäß der SEQ ID NO: 1-3, bzw. D3r, Drp, D3a bzw. D3(p2k) gemäß der SEQ ID NO: 9-12 sind dann an der Position 12 am freien C-terminus amidiert. Polymere, wie beispielweise RD2D3, D3RD2, D3D3, RD2RD2 oder DB3DB3 sind an Position 24 am freien C-terminus amidiert.

Die erfindungsgemäßen Peptide gemäß der SEQ ID NO: 4, 5 und 6, sowie die nicht erfindungsgemäßen Peptide der SEQ ID: 1, 2, 3, 7, 8, 9, 10, 11 und 12 sind in einer weiteren Ausgestaltung am freien C-terminus mit dem freien N-terminus kovalent miteinander verbunden und liegen dann entsprechend zyklisiert vor. Auch durch den Ringschluss wird vorteilhaft bewirkt, dass die Carboxylgruppe am freien C-terminus nicht mehr vorhanden ist.

Das erfindungsgemäße Peptid weist vorteilhaft eine Aminosäuresequenz auf, bei der die Zyklisierung des linearen Moleküls z. B. durch eine kovalente Bindung der ersten mit der letzten Aminosäure, z.B. über eine Kondensationsreaktion, erfolgt ist. Selbstverständlich existieren weitere Möglichkeiten der Zyklisierung, z. B. indem andere Aminosäuren miteinander verknüpft werden. Lediglich beispielhaft sei die Verknüpfung der zweiten Aminosäure mit der letzten Aminosäure genannt. Genauso denkbar ist jede mögliche andere Verknüpfung.

Im Falle, dass die erste und die letzte Aminosäure des Peptids miteinander verknüpft werden, wird vorteilhaft bewirkt, dass keine offenen Enden in der Peptidkette (Aminosäuresequenz) vorliegen.

Diese Maßnahme bewirkt ferner, dass alle Peptide mit linearen Aminosäuresequenzen, die nach der Zyklisierung die gleiche, nicht mehr unterscheidbare Aminosäure-Reihenfolge ergeben, in diesem Sinne identisch sind.

Beispiel: Die lineare Aminosäuresequenz des bekannten Peptids D3 ist rprtrlhthrnr. Das entsprechende durch eine Amidbindung zwischen der N-terminalen Aminogruppe und der C-terminalen Carboxylgruppe verknüpfte, zyklisierte Peptid "cD3" ist nicht mehr zu unterscheiden von den zyklisierten Peptiden prtrlhthrnrr, rtrlhthrnrrp, trlhthrnrrpr, rlhthrnrrprt, Ihthrnrrprtr, hthrnrrprtrl, thrnrrprtrlh, hrnrrprtrlht, rnrrprtrlhth, nrrprtrlhthr, oder rrprtrlhthrn. Aus jeder dieser Sequenzen lässt sich das cD3 weiterhin auch ableiten.

Die erfindungsgemäß beanspruchten Effekte der höheren Affinität und Effektivität treten darüber hinaus gegenüber einem, vorzugsweise sogar jedem linearen, bindenden Peptid auf, aus dem sich ein zyklisiertes bzw. anderweitig modifiziertes, erfindungsgemäßes Peptid ableiten lässt.

Die Herstellung zyklisierter Peptide ist im Übrigen Stand der Technik, und kann beispielweise nach dem Verfahren wie es in DE 102005049537 A1 beschrieben ist, erfolgen.

Vorteilhaft bewirkt die Zyklisierung über die erste und letzte Aminosäure des Peptids, dass keine "offenen" Enden der Peptidkette mehr existieren, die oft Angriffspunkte für Peptidabbauende Aktivitäten in Zellen, Tieren oder Menschen sind, z. B. durch Aminopeptidasen und Carboxypeptidasen.

Mittels zyklisierter Monomere, wie z. B. cD3, cRD2 und so weiter bzw. zyklisierter Polymere wie z. B. cRD2D3, cD3D3, cDB3DB3 und so weiter wird zusätzlich vorteilhaft bewirkt, dass diese zyklisierten Peptide als Seiteneffekt unter Umständen auch nicht leicht abgebaut werden, wenngleich dieser Effekt nicht ausschlaggebend ist. Er gilt im Übrigen, wie gezeigt wurde, auch nur für den Fall einer head-to-tail- oder tail-to-head-Zyklisierung, bei dem entsprechend beide Enden des linearen Peptids miteinander verknüpft werden.

In einer weiteren Ausgestaltung sind die Polymere aus identischen Monomeren, wie z. B. D3, RD2 oder DB3 aufgebaut oder aus einer Kombination von 2, 3, 4, 5, 6, 7, 8, 9, 10 unterschiedlichen, verschiedenen der oben genannten Monomere, als sogenannte Kombinations-Polymere. Die Monomere können auch teilweise identisch sein. Die Anzahl der identischen Monomere in den Kombinations-Polymeren ist frei wählbar.

Polymere können beispielsweise über chemische Synthese bzw. Peptidsynthese hergestellt werden.

In einer Ausführung der Erfindung sind die Monomere kovalent miteinander verknüpft. In einer weiteren Ausführung sind die Monomere nicht kovalent miteinander verbunden.

Eine kovalente Verbindung bzw. Verknüpfung der Monomer-Einheiten liegt im Sinne der Erfindung vor, falls die Peptide Kopf an Kopf, Schwanz an Schwanz oder Kopf an Kopf linear miteinander verknüpft werden, mit oder ohne dazwischen eingefügte Linker oder Linker-Gruppen.

Eine nicht kovalente Verknüpfung im Sinne der Erfindung liegt vor, falls die Monomere beispielsweise über Biotin und Streptavidin, insbesondere Streptavidin-Tetramer miteinander verknüpft sind.

In einer Variante der vorliegenden Erfindung können die Monomere linear miteinander verknüpft sein, insbesondere wie oben beschrieben. In einer anderen Variante sind die Monomere verzweigt miteinander zu dem erfindungsgemäßen Polymer verknüpft.

Bei einem verzweigten Polymer kann es sich erfindungsgemäß um ein Dendrimer handeln, bei dem die Monomere kovalent oder nicht kovalent miteinander verknüpft sind.

Alternativ können die Monomere auch mit einem Plattform-Molekül (wie z.B. PEG oder Zucker) verknüpft sein und so ein verzweigtes Polymer bilden.

Alternativ sind auch Kombinationen dieser Optionen möglich.

Monomere und Polymere werden im Folgenden als erfindungsgemäße Peptide bezeichnet.

In einer nicht erfindungsgemäßen Variante der Offenbarung handelt es sich um ein Peptid mit der Aminosäuresequenz gemäß SEQ ID NO: 1, SEQ ID NO: 2, SEQ ID NO: 3, , SEQ ID NO: 7, SEQ ID NO: 8, SEQ ID NO: 9, SEQ ID NO: 10, SEQ ID NO: 11, und / oder SEQ ID NO: 12 und/oder Homologe davon mit einer Identität von 50%.

"Homologe Sequenzen" oder "Homologe" bedeutet im Sinne der Erfindung, dass eine Aminosäuresequenz eine Identität mit einer der oben genannten Aminosäuresequenz der Monomere von mindestens 50, 55, 60, 65, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 ,100 % aufweist. Anstelle des Begriffs "Identität" werden in der vorliegenden Beschreibung die Begriffe "homolog" oder "Homologie" gleichbedeutend verwendet. Die Identität zwischen zwei Nukleinsäuresequenzen oder Polypeptidsequenzen wird durch Vergleich mit Hilfe des Programms BESTFIT basierend auf dem Algorithmus von Smith, T.F. und Waterman, M.S (Adv. Appl. Math. 2: 482-489 (1981)) berechnet unter Einstellung folgender Parameter für Aminosäuren: Gap creation penalty: 8 und Gap extension penalty: 2; und folgender Parameter für Nukleinsäuren: Gap creation penalty: 50 und Gap extension penalty: 3. Bevorzugt wird die Identität zwischen zwei Nukleinsäuresequenzen oder Polypeptidsequenzen durch die Identität der Nukleinsäuresequenz / Polypeptidequenz über die jeweils gesamte Sequenzlänge definiert, wie sie durch Vergleich mit Hilfe des Programms GAP basierend auf dem Algorithmus von Needleman, S.B. und Wunsch, C.D. (J. Mol. Biol. 48: 443-453) unter Einstellung folgender Parameter für Aminosäuren berechnet wird: Gap creation penalty: 8 und Gap extension penalty: 2; und die folgenden Parameter für Nukleinsäuren Gap creation penalty: 50 und Gap extension penalty: 3.

Zwei Aminosäuresequenzen sind im Sinne der vorliegenden Erfindung identisch wenn sie dieselbe Aminosäuresequenz besitzen.

Unter Homologe sind in einer Variante die entsprechenden retro-inversen Sequenzen der oben genannten Monomere zu verstehen. Mit dem Begriff "retro-inverse Sequenz" wird erfindungsgemäß eine Aminosäuresequenz bezeichnet, die sich aus Aminosäuren in der enantiomeren Form zusammensetzt (invers: Chiralität des alpha-C-Atoms invertiert) und bei der zusätzlich die Sequenzreihenfolge zur ursprünglichen Aminosäuresequenz umgekehrt wurde (retro = rückwärts).

In einer weiteren Variante binden die erfindungsgemäßen Peptide an Teile des Amyloid Beta-Peptids.

In einer weiteren nicht erfindungsgemäßen Variante weisen die Peptide der Offenbarung Sequenzen auf, die sich von den angegebenen Sequenzen um bis zu drei Aminosäuren unterscheiden.

Ferner werden als Peptide auch Sequenzen eingesetzt, die die oben genannten Sequenzen enthalten.

In einer weiteren nicht erfindungsgemäßen Variante weisen die Peptide Fragmente der oben genannten Sequenzen auf oder weisen homologe Sequenzen zu den oben genannten Sequenzen auf.

Erfindungsgemäß handelt es sich um ein Peptid zur Verwendung in der Medizin, bevorzugt zur Behandlung der Morbus Alzheimer.

In einer Ausführung der vorliegenden Erfindung besteht das Peptid im Wesentlichen aus D-Aminosäuren.

Im Sinne der vorliegenden Erfindung bedeutet der Begriff "im Wesentlichen aus D-enantiomeren Aminosäuren", dass die erfindungsgemäß einzusetzenden Monomere mindestens 50%, 60%, bevorzugt 75%, 80%, besonders bevorzugt 85%, 90%, 95%, insbesondere 96%, 97%, 98%, 99%, 100% aus D-enantiomeren Aminosäuren aufgebaut sind.

In einer Ausführung der vorliegenden Erfindung handelt es sich bei den erfindungsgemäßen Monomer-Peptiden um Derivate des D-enantiomeren D-Peptids D3. Derivate im Sinne der Offenbarung sind von D3 abgeleitete Peptid-Sequenzen, die nach einem der drei folgenden Verfahren erzielt werden:
a) Änderung der Reihenfolge und/oder Anzahl der Aminosäurebausteine in D3. Es werden dabei nur Aminosäuren eingesetzt, die in der D3-Sequenz vorhanden sind.
b) Deletion von 1, 2, 3, 4, 5, 6, 7, 8, 9, 10 oder 11 Aminosäuren der D3-Sequenz.
c) Austausch von 1, 2, 3, 4, 5 oder 6 Aminosäuren durch andere Aminosäuren, bevorzugt D-Enantiomere.

In einer weiteren Variante handelt es sich um ein erfindungsgemäßes Peptid zur Hemmung der Fibrillenbildung von Amyloid-Beta-Oligomeren. Die erfindungsgemäßen Peptide enttoxifizieren die A-Beta-Oligomere oder daraus gebildete Polymere, sowie Fibrillen, indem sie daran binden und so in nicht toxische Verbindungen überführen. Demgemäß ist Gegenstand der vorliegenden Erfindung auch ein Verfahren zur Enttoxifizierung der A-Beta-Oligomeren, daraus gebildeten Polymeren oder Fibrillen.

Gegenstand der Erfindung sind in einer Ausführung auch erfindungsgemäße Peptide, die mit einer weiteren Substanz verknüpft sind.

Bei der Verknüpfung handelt es sich im Sinne der Erfindung um eine chemische Bindung wie sie in Römpp Chemie Lexikon, 9. Auflage, Band 1, Seite 650 ff, Georg Thieme Verlag Stuttgart definiert ist, bevorzugt um eine Hauptvalenz Bindung, insbesondere eine kovalente Bindung.

Bei den Substanzen handelt es sich in einer Variante um Arzneimittel oder Wirkstoffe, definiert gemäß Arzneimittelgesetz §2 beziehungsweise. §4 (19), Stand September 2012. In einer Alternative sind Wirkstoffe therapeutisch aktive Stoffe die als arzneilich wirksame Stoffe verwendet werden. Bevorzugt werden Entzündungshemmer eingesetzt.

Bei den Substanzen handelt es sich in einer weiteren Variante um Verbindungen die die Wirkung der Peptide verstärken.

In einer Alternative sind solche Verbindungen Aminopyrazol und/oder Aminopyrazolderivate. Aminopyrazolderivate im Sinne der Erfindung ist 3-Aminopyrazol-5-carbonsäure oder 3-Nitropyrazol-5-carbonsäure sowie alle Abkömmlinge, in denen die heterocyclische CH-Gruppe gegen -CR- oder -N- oder -O- oder -S- ausgetauscht wurde, sowie alle daraus abgeleiteten peptidischen Dimere, Trimere oder -Tetramere, bevorzugt Aminopyrazol-Trimer.

In einer weiteren Alternative handelt es sich um Verbindungen, die die Löslichkeit der Peptide und/oder die Passage der Blut-Hirn-Schranke verbessern.

In einer Alternative haben die Peptide erfindungsgemäß jede beliebige Kombination von mindestens zwei oder mehr Merkmalen der oben beschriebenen Varianten, Ausführungen und/oder Alternativen.

Im Rahmen der Erfindung wurde weiterhin erkannt, dass die erfindungsgemäß modifizierten Peptide im Vergleich zu linearen, bindenden Peptiden, bei denen der freie C-terminus, also die C-terminale Carboxylguppe, nicht modifiziert ist und entsprechend eine negative Ladung trägt, mit einer höheren Affinität an Abeta-Spezies binden, und zwar insbesondere auch an die besonders toxischen Amyloid-Beta-Oligomere. Das heißt, dass der K_{D}-Wert bei den erfindungsgemäßen Peptiden niedriger ist, als bei linearen Peptiden, bei denen der freie C-terminus, also die C-terminale Carboxylguppe, nicht modifiziert ist und entsprechend eine negative Ladung trägt.

In einer weiteren bevorzugten Ausgestaltung der Erfindung ist daher die Affinität der Bindung der erfindungsgemäß modifizierten Peptide ohne negative Ladung am C-terminus im Vergleich zu linearen Peptiden mit negativer Ladung am C-terminus aber im Übrigen gleicher Aminosäuresequenz, um 1%, 2, 3, 4, 5, 6, 7, 8, 9, insbesondere 10%, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, insbesondere 100%, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, insbesondere 200 %, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, insbesondere 300%, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391, 392, 393, 394, 395, 396, 397, 398, 399, insbesondere 400%, 401, 402, 403, 404, 405, 406, 407, 408, 409, 410, 411, 412, 413, 414, 415, 416, 417, 418, 419, 420, 421, 422, 423, 424, 425, 426, 427, 428, 429, 430, 431, 432, 433, 434, 435, 436, 437, 438, 439, 440, 441, 442, 443, 444, 445, 446, 447, 448, 449, 450, 451, 452, 453, 454, 455, 456, 457, 458, 459, 460, 461, 462, 463, 464, 465, 466, 467, 468, 469, 470, 471, 472, 473, 474, 475, 476, 477, 478, 479, 480, 481, 482, 483, 484, 485, 486, 487, 488, 489, 490, 491, 492, 493, 494, 495, 496, 497, 498, 499, vorteilhaft sogar 500%, 501, 502, 503, 504, 505, 506, 507, 508, 509, 510, 511, 512, 513, 514, 515, 516, 517, 518, 519, 520, 521, 522, 523, 524, 525, 526, 527, 528, 529, 530, 531, 532, 533, 534, 535, 536, 537, 538, 539, 540, 541, 542, 543, 544, 545, 546, 547, 548, 549, 550, 551, 552, 553, 554, 555, 556, 557, 558, 559, 560, 561, 562, 563, 564, 565, 566, 567, 568, 569, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599, besonders vorteilhaft 600%, 601, 602, 603, 604, 605, 606, 607, 608, 609, 610, 611, 612, 613, 614, 615, 616, 617, 618, 619, 620, 621, 622, 623, 624, 625, 626, 627, 628, 629, 630, 631, 632, 633, 634, 635, 636, 637, 638, 639, 640, 641, 642, 643, 644, 645, 646, 647, 648, 649, 650, 651, 652, 653, 654, 655, 656, 657, 658, 659, 660, 661, 662, 663, 664, 665, 666, 667, 668, 669, 670, 671, 672, 673, 674, 675, 676, 677, 678, 679, 680, 681, 682, 683, 684, 685, 686, 687, 688, 689, 690, 691, 692, 693, 694, 695, 696, 697, 698, 699, besonders vorteilhaft 700%, 701, 702, 703, 704, 705, 706, 707, 708, 709, 710, 711, 712, 713, 714, 715, 716, 717, 718, 719, 720, 721, 722, 723, 724, 725, 726, 727, 728, 729, 730, 731, 732, 733, 734, 735, 736, 737, 738, 739, 740, 741, 742, 743, 744, 745, 746, 747, 748, 749, 750, 751, 752, 753, 754, 755, 756, 757, 758, 759, 760, 761, 762, 763, 764, 765, 766, 767, 768, 769, 770, 771, 772, 773, 774, 775, 776, 777, 778, 779, 780, 781, 782, 783, 784, 785, 786, 787, 788, 789, 790, 791, 792, 793, 794, 795, 796, 797, 798, 799, ebenfalls besonders vorteilhaft 800%, 801, 802, 803, 804, 805, 806, 807, 808, 809, 810, 811, 812, 813, 814, 815, 816, 817, 818, 819, 820, 821, 822, 823, 824, 825, 826, 827, 828, 829, 830, 831, 832, 833, 834, 835, 836, 837, 838, 839, 840, 841, 842, 843, 844, 845, 846, 847, 848, 849, 850, 851, 852, 853, 854, 855, 856, 857, 858, 859, 860, 861, 862, 863, 864, 865, 866, 867, 868, 869, 870, 871, 872, 873, 874, 875, 876, 877, 878, 879, 880, 881, 882, 883, 884, 885, 886, 887, 888, 889, 890, 891, 892, 893, 894, 895, 896, 897, 898, 899, ebenfalls besonders vorteilhaft 900%, 901, 902, 903, 904, 905, 906, 907, 908, 909, 910, 911, 912, 913, 914, 915, 916, 917, 918, 919, 920, 921, 922, 923, 924, 925, 926, 927, 928, 929, 930, 931, 932, 933, 934, 935, 936, 937, 938, 939, 940, 941, 942, 943, 944, 945, 946, 947, 948, 949, 950, 951, 952, 953, 954, 955, 956, 957, 958, 959, 960, 961, 962, 963, 964, 965, 966, 967, 968, 969, 970, 971, 972, 973, 974, 975, 976, 977, 978, 979, 980, 981, 982, 983, 984, 985, 986, 987, 988, 989, 990, 991, 992, 993, 994, 995, 996, 997, 998, 999, oder sogar um 1000 %, oder sogar um 10000% oder sogar um bis zu 100000% oder 1000000% erhöht, wobei jeder Zwischenwert angenommen werden kann. Dies betrifft insbesondere aber nicht ausschließlich eine erhöhte Affinität zu der high affinity site des A-Beta (Monomer, Oligomer, Fibrillen und so weiter).

Dies wird durch einen entsprechend erniedrigten K_{D}-Wert angezeigt. Der K_{D}-Wert als Maß für die Affinität der Bindung des erfindungsgemäß modifizierten Peptids an Amyloid-Beta-Spezies und insbesondere an Amyloid-Beta-Oligomer ist im Vergleich zu einem linearen, bindenden Peptid mit negativer Ladung am freien C-terminus, um 1%, 2, 3, 4, 5, 6, 7, 8, 9, insbesondere 10%, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, insbesondere 99,1, 99,2, 99,3, 99,4, 99,5%, 99,6, 99,7, 99,8, 99,9 bis zu 99,99 oder sogar 99,999% erniedrigt, wobei jeder Zwischenwert angenommnen werden kann.

Diese niedrigeren K_{D}-Werte beziehen sich vorteilhaft insbesondere aber nicht ausschließlich auf die high affinity site von A-Beta-Spezies (Monomer, Oligomer, Fibrillen und so weiter).

Erfindungsgemäß modifizierte Peptide können daher effizienter als Sonden für diagnostische Zwecke verwendet werden, als lineare, bindende Peptide mit negativer Ladung am freien C-terminus, insbesondere auch effizienter als ihre linearen Peptid-Pendants mit identischer Aminosäuresequenz verwendet werden.

Sie können aber insbesondere auch effizienter als Therapeutika verwendet werden, als lineare, bindende Peptide mit negativer Ladung am freien C-terminus, insbesondere effizienter als ihre linearen Peptid-Pendants mit identischer Aminosäuresequenz.

Im unmittelbaren Vergleich eines erfindungsgemäß modifizierten Peptids zu einem Peptid mit negativer Ladung am C-terminus schneidet das erfindungsgemäße Peptid besser in Hinblick auf Affinität und Effektivität ab.

Im Rahmen der Erfindung wurde nämlich weiterhin erkannt, dass die erfindungsgemäß modifizierten Peptide im Vergleich zu Peptiden mit negativer Ladung am freien C-terminus, insbesondere aber im Vergleich zu ihren Peptid-Pendants mit identischer Aminosäuresequenz, zusätzlich auch mit einer höheren Effektivität bzw. Effizienz die Bildung besonders toxischer Amyloid-Beta-Oligomere verhindern oder deren Zerstörung und / oder Endtoxifizierung bewirken. Diese Effektivität ist insbesondere um 1%, 2, 3, 4, 5, 6, 7, 8, 9, insbesondere 10%, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 99,9, besonders vorteilhaft sogar um 100% erhöht.

Eine Probe mit den verschiedenen Aß-Konformeren wird hierzu testweise im einfachsten Fall z. B. fraktioniert. In jeder Fraktion werden entsprechend des Fraktionierungsschritts verschiedene Konformere, wie Monomere, Oligomere, Fibrillen oder höhere Aggregate angereichert und können sodann exakt bestimmt werden.

Der Begriff "exakt bestimmt" umfasst einen Kalibrierungsschritt bei der Fraktionierung durch Moleküle bekannter Art und Verhaltens. Nach der Fraktionierung liegt in jeder Fraktion nur eine bestimmte Art an Konformeren des Aß vor, z. B. Monomere, Oligomere oder Fibrillen und so weiter.

Beispielsweise werden die Konformere bei einer Dichtegradientenzentrifugation als Fraktionierungsschritt nach ihrem s-Wert oder Sedimentationskoeffizienten aufgetrennt. Moleküle unterschiedlicher Größe können einen identischen hydrodynamischen Radius besitzen, haben aber dennoch unterschiedliche s-Werte und werden danach auch aufgetrennt. Durch eine Kalibration mit Molekülen von bekanntem s-Wert sind die mittels Dichtegradientenzentrifugation erhaltenen Aß Konformere exakt nach ihrem s-Wert bestimmt.

Im Weiteren werden die erhaltenen Fraktionen mit und ohne Wirkstoff behandelt und z. B. durch RP-HPLC bestimmt. Auf diese Weise kann man die Effektivität des Wirkstoffs bestimmen.

Ein weiteres Verfahren wird nachfolgend vorgestellt. Zur quantitativen Analyse von Wirkstoffen kann der sogenannte QIAD-Test eingesetzt (quantitative determination of interference with Aβ aggregate size distribution). Das Verfahren zur quantitativen Analyse des Einflusses eines Wirkstoffs auf die Partikelgrößenverteilung amyloider Peptide und / oder Proteine in einer Probe, weist dabei nachfolgende Schritte auf. Zunächst lässt man A-Beta unter kontrollierten Bedingungen aggregieren, so dass je verschiedene A-Beta-Aggregate entstehen. Die Bedingungen werden so gewählt, dass besonders viele, kleine, besonders cytotoxische ABeta-Oligomere gebildet wurden. Als nächstes wird die zu untersuchende Substanz, z.B. eines der erfindungsgemäßen modifizierten Peptide zu der Probe zugegeben. Der Wirkstoff ändert die Partikelgrößenverteilung in der Probe. Diese Änderung wird quantitativ festgestellt. Die Änderung ist ein Maß für die Reduktion oder sogar für die vollständige Eliminierung bestimmter toxischer Spezies einer bestimmten Partikelgröße. Durch das QIAD-Verfahren wird die Zunahme oder die Abnahme von A-Beta-Aggregaten einer bestimmten Partikelgröße gemessen. Während einige A-Beta-Aggregate mit einer bestimmten Größe anfänglich in der Probe vorhanden waren, werden diese unter Einfluss des Wirkstoffs reduziert oder sogar vollständig eliminiert. Andere Partikelgrößen nehmen unter dem Einfluss des Wirkstoffs zu oder bleiben konstant. Die Partikel, die aus dem A-Beta gebildet sind, werden vorzugsweise nach ihrem hydrodynamischen Radius der Partikel voneinander getrennt. Auf diese Weise wird vorteilhaft bewirkt, dass aus der Probe eine Mehrzahl an Fraktionen erhalten wird. In den Fraktionen sind die Partikel an amyloiden Peptiden und / oder Proteinen mit einer bestimmten Aggregatgröße angereichert. Diese Trennung der Partikel kann mittels einer Dichtegradientenzentrifugation vorgenommen werden. Die Fraktionen werden räumlich voneinander getrennt, z. B. durch Abpipettieren. Abschließend wird die Konzentration an A-Beta in der jeweiligen Fraktion durch eine vollständige Denaturierung der A-Beta-Spezies während einer im Anschluss an die Fraktionierung durchgeführten Umkehrphasen (RP-) HPLC bestimmt. Die Denaturierung der Aggregate kann z. B. mit 30 % Acetonitril und 0,1 % Trifluoressigsäure bei einer Säulentemperatur von 80 °C vollständig erfolgen und auf einer C8-Säule nach Hydrophobizität aufgetrennt werden. Eluierendes A-Beta wird mittels der UV-Absorption bei 215 nm detektiert. Die Peakflächenintegration kann mittels Agilent Chemstation Software erfolgen. Die Verrechnung daraus entstandener Werte mit einer zuvor durchgeführten Kalibrierung erlaubt die Berechnung der Konzentration des in der jeweiligen Fraktion vorhandenen A-Beta. Je Fraktion kann der Mittelwert aus mehreren z. B. sechs voneinander unabhängig durchgeführten Experimenten mit der sich ergebenen Standardabweichung berechnet werden. Der Vorteil der HPLC-Analyse liegt darin, dass man unabhängig vom Aggregationszustand und eines Lösungsmittels sehr sensitiv detektieren (z. B. ca. 20 nM oder 1,8 ng Aß1-42) und zuverlässig quantifizieren kann. Ein entscheidender Vorteil des Verfahrens besteht in der Kopplung von Dichtegradientenzentrifugation und Umkehrphasen HPLC, welche eine zuverlässige Quantifizierung auch von Aß-Oligomeren möglich macht.

Der erfindungsgemäße Effekt einer gesteigerten Effektivität der Eliminierung (bzw. der Bildung) von Amyloid-Beta-Spezies und insbesondere Amyloid-Beta-Oligomeren kann mit einem dieser Verfahren, aber nicht ausschließlich mit diesen Verfahren erfolgen.

In einer besonders bevorzugten Ausgestaltung der Erfindung treten die genannten Effekte der gesteigerten Affinität und Effektivität der Eliminierung, Enttoxifizierung (bzw. der Bildung) auch *in vitro* und / oder *in vivo* auf.

Gegenstand der Erfindung ist auch ein erfindungsgemäßes Peptid zur Bindung an aggregierte A-Beta-Peptide.

Weiterer Gegenstand der Erfindung ist ein Verfahren zur Herstellung des erfindungsgemä-ßen Peptids durch Peptidsynthese, wie zum Beispiel dem Fachmann bekannt, organische Synthesemethoden für beliebige Verbindungen mit geringem Molekulargewicht (lowmolecular weight compounds).

Die Erfindung betrifft ferner auch eine Zusammensetzung enthaltend das erfindungsgemäße Peptid, insbesondere zur Behandlung von Morbus Alzheimer.

Gegenstand der vorliegenden Erfindung ist ferner eine Zusammensetzung enthaltend das erfindungsgemäße Peptid, insbesondere zur Verhinderung von toxischen A-Beta-Oligomeren, oder zur Zerstörung von daraus gebildeten Polymeren oder Fibrillen.

Die erfindungsgemäße "Zusammensetzung" kann z. B. ein Impfstoff, ein Medikament (z. B. in Tablettenform), eine Injektionslösung, ein Nahrungs- oder Nahrungsergänzungsmittel sein, enthaltend das erfindungsgemäße Peptid in einer aufgrund des Fachwissens herzustellenden Formulierung.

Die Erfindung betrifft ferner auch ein Kit enthaltend das erfindungsgemäße Peptid.

In einem solchen Kit können die erfindungsgemäßen Peptide in Behältern ggf. mit/in Puffern oder Lösungen verpackt sein. Alle Komponenten des Kits können in demselben Behälter oder getrennt voneinander verpackt sein. Ferner kann das Kit Anweisungen für dessen Gebrauch enthalten. Ein solches Kit kann beispielsweise die erfindungsgemäßen in einer Injektionsflasche mit Stopfen und/oder Septum enthalten. Ferner kann darin beispielsweise auch eine Einmal-Spritze enthalten sein.

Ein weiterer Gegenstand der vorliegenden Erfindung ist die Verwendung des erfindungsgemäßen Peptids als Sonde zur Identifizierung, qualitativen und/oder quantitativen Bestimmung von Amyloid-Beta-Oligomeren oder Fibrillen.

Gegenstand der vorliegenden Erfindung ist auch eine Sonde, enthaltend das erfindungsgemäße Peptid zur Identifizierung, qualitativen und/oder quantitativen Bestimmung von Amyloid-Beta-Oligomeren.

Solche Sonden sind von großer Bedeutung, da damit eine frühe Diagnose der AD möglich wird. Mit der Frühdiagnose kann der Krankheit schon in einem sehr frühen Stadium entgegengewirkt werden.

Solche molekularen Sonden enthalten das erfindungsgemäße Polymer und gegebenenfalls Farbstoffe, Fluoreszenzfarbstoffe, radioaktive Isotope, (PET etc.), Gadolinium (MRI), sowie alternative Stoffe geeignet für die Bildgebung der Sonden und können den Patienten z. B. intravenös injiziert werden. Nach Passage über die Bluthirnschranke können die Sonden an A-Beta-Oligomere und/oder Plaques binden. Die so markierten A-Beta-Oligomere und/oder Plaques können mittels bildgebender Verfahren wie z.B. SPECT, PET, CT, MRT, Protonen-MR-Spektroskopie usw. sichtbar gemacht werden.

Ferner betrifft die Erfindung auch das Peptid zur Verwendung bei der Verhinderung von Amyloid-Beta-Oligomeren und/oder Amyloid-Beta-Peptid-Aggregaten und/oder Amyloid-Beta-Fibrillen.

Erfindungsgemäß ist auch ein Peptid zur Verwendung bei der Enttoxifizierung von toxischen Amyloid-Beta-Oligomeren und/oder Aggregaten. Insbesondere wird es verwendet, um an Amyloid-Beta-Oligomere und/oder Aggregate zu binden und so amorphe, nicht toxische Aggregate zu bilden.

Es wurde erkannt, dass wenn bereits Aß-Oligomere vorhanden sind, es das Ziel einer Behandlung sein muss, diese durch Substanzen zu adressieren, die eine möglichst hohe Affinität zu A-Beta besitzen. De facto kann die Affinität gar nicht groß genug sein und die entsprechende Dissoziationskonstante des erfindungsgemäßen Peptids liegt dann im sub-µM-Bereich, besser noch pM-Bereich oder noch tiefer.

Es wurde im Rahmen der Erfindung erkannt, dass A-Beta-Monomere, als Bausteine der ABeta-Oligomere, ständig im menschlichen Körper entstehen und vermutlich *per se* nicht toxisch sind. Es besteht sogar die Möglichkeit, dass Monomere eine positive Funktion besitzen. A-Beta-Monomere können sich in Abhängigkeit von ihrer Konzentration zufällig zusammen lagern. Die Konzentration ist abhängig von ihrer Bildungs- und Abbaurate im Körper. Findet mit zunehmendem Alter eine Erhöhung der Konzentration an A-Beta-Monomeren im Körper statt, ist eine spontane Zusammenlagerung der Monomere zu A-Beta-Oligomeren immer wahrscheinlicher. Die so entstandenen A-Beta-Oligomere könnten sich analog zu den Prionen vermehren und letztendlich zur Morbus Alzheimer-Krankheit führen.

Es wurde ferner erkannt, dass ein wichtiger Unterschied zwischen Prävention und Behandlung oder gar Heilung der AD in der Tatsache liegt, dass eine Prävention möglicherweise schon durch die Verhinderung der Bildung der ersten A-Beta-Oligomere erreicht werden kann. Hierzu sind einige, wenige A-Beta-Liganden ausreichend, die wenig affin und selektiv bezüglich der A-Beta-Oligomere sind.

Die Bildung der A-Beta-Oligomere aus vielen Monomeren ist eine Reaktion hoher Ordnung und damit in hoher Potenz von der A-Beta-Monomer-Konzentration abhängig. Somit führt schon eine kleine Verringerung der aktiven A-Beta-Monomer-Konzentration zu einer Verhinderung der Bildung der ersten A-Beta-Oligomere. Auf diesem Mechanismus basieren vermutlich die bisher sich in der Entwicklung befindlichen eher präventiven Therapie-Konzepte und Substanzen.

Bei der Behandlung von AD ist jedoch von einer völlig veränderten Situation auszugehen. Hier liegen A-Beta-Oligomere oder evtl. auch schon größere Polymere oder Fibrillen vor, die sich durch Prionen-ähnliche Mechanismen vermehren. Diese Vermehrung ist jedoch eine Reaktion niederer Ordnung und ist kaum noch von der A-Beta-Monomer-Konzentration abhängig.

Sind also A-Beta-Oligomere bereits entstanden, muss es das Ziel einer Behandlung sein, diese durch Substanzen zu adressieren, die eine möglichst hohe Affinität zu Aß-Oligomeren besitzen und / oder diese besonders effizient eliminieren und / oder die Bildung besonders effizient verhindern bzw. diese enttoxifizieren. Die entsprechende Dissoziationskonstante müsste dazu im sub-µM, nM oder pM-Bereich oder noch tiefer liegen.

Diese Anforderungen in Hinblick auf Diagnose (Sonden) und Therapie der Alzheimerschen Demenz sind mit der Bereitstellung der erfindungsgemäßen Peptide erfüllt. Die erfindungsgemäßen Peptide binden im Sinne der Diagnose und/oder Therapie die Amyloid-Beta-Spezies und insbesondere die A-Beta-Oligomere mit entsprechend niedriger Dissoziationskonstante. Weiterer Gegenstand sind entsprechend die erfindungsgemäßen Peptide zur Verwendung als Therapeutikum von Morbus Alzheimer.

Die erfindungsgemäßen Peptide binden besonders gut an A-Beta-Oligomere, insbesondere an lösliche A-Beta-Oligomere.

Eine besonders starke Bindung der erfindungsgemäßen Peptide an die Zielmoleküle wird durch eine hohe Spezifität und/oder Affinität zu dem Zielmolekül der erfindungsgemäßen Peptide hervorgerufen. Die gebildeten Komplexe haben also eine geringe Dissoziationskonstante (KD-Wert).

Mittels des Thioflavin-T-Tests konnte gezeigt werden, dass die Peptide der Offenbarung die Fibrillenbildung von A-Beta-Peptiden sehr effizient hemmen, besonders die Peptide mit den SEQ ID NO: 1 - 12, insbesondere die erfindungsgemäßen Peptide der SEQ ID NO: 4, SEQ ID NO:5 und SEQ ID NO:6 aber auch die nicht erfindungsgemäßen Peptide der SEQ ID NO: 7 und SEQ ID NO: 8.

Weiterer Gegenstand ist die Verwendung der erfindungsgemäßen Peptide in einem Verfahren zur Behandlung (in vitro, ex vivo) von Blut, Blutprodukten und/oder Organen, dadurch gekennzeichnet, dass das Blut, die Blutprodukte und/oder Organe dem menschlichen oder tierischen Körper entnommen werden und A-(Amyloid)-Beta-Oligomere entfernt und/oder enttoxifiziert werden.

### Ausführungsbeispiele

Im Weiteren wird die Erfindung an Hand von Ausführungsbeispielen und der beigefügten Figuren näher erläutert, ohne dass es hierdurch zu einer Beschränkung der Erfindung kommen soll.

Es zeigen:
- Figur 1:: Object recognition test
- Figur 2:: RotaRod
- Figur 3:: SHIRPA
- Figur 4:: Morris water maze
- Figur 5:: Object recognition
- Figur 6:: K_{D}-Werte (Kinetik) zu Amyloid-Beta-Monomer
- Figur 7:: K_{D}-Werte (Kinetik) zu Amyloid-Beta-Oligomer
- Figur 8:: K_{D}-Werte (Kinetik) zu Amyloid-Beta-Fibrillen

Zur quantitativen Analyse von Wirkstoffen wurde der QIAD-Test eingesetzt (quantitative determination of interference with Aβ aggregate size distribution). Das Verfahren zur quantitativen Analyse des Einflusses eines Wirkstoffs auf die Partikelgrößenverteilung amyloider Peptide und / oder Proteine in einer Probe, weist dabei nachfolgende Schritte auf: Zunächst lässt man A-Beta unter kontrollierten Bedingungen aggregieren, so dass je verschiedene A-Beta-Aggregate entstehen. Für das in Tabelle 1 zusammengefasste Ergebnis wurden die Bedingungen so gewählt, dass besonders viele, kleine, besonders cytotoxische A-Beta-Oligomere gebildet wurden. Als nächstes wird die zu untersuchende Substanz, z.B. eines der oben genannten D-enantiomeren Peptide zu der Probe zugegeben. Der Wirkstoff ändert die Partikelgrößenverteilung in der Probe. Diese Änderung wird quantitativ festgestellt. Die Änderung ist ein Maß für die Reduktion oder sogar für die vollständige Eliminierung bestimmter toxischer Spezies einer bestimmten Partikelgröße. Durch das QIAD-Verfahren wird also die Zunahme oder die Abnahme von A-Beta-Aggregaten einer bestimmten Partikelgröße gemessen. Während einige A-Beta-Aggregate mit einer bestimmten Größe anfänglich in der Probe vorhanden waren, werden diese unter Einfluss des Wirkstoffs reduziert oder sogar vollständig eliminiert. Andere Partikelgrößen nehmen unter dem Einfluss des Wirkstoffs zu oder bleiben konstant. Die Partikel, die aus dem A-Beta gebildet sind, werden vorzugsweise nach ihrem hydrodynamischen Radius der Partikel voneinander getrennt. Auf diese Weise wird vorteilhaft bewirkt, dass aus der Probe eine Mehrzahl an Fraktionen erhalten wird. In den Fraktionen sind die Partikel an amyloiden Peptiden und / oder Proteinen mit einer bestimmten Aggregatgröße angereichert. Diese Trennung der Partikel kann mittels einer Dichtegradientenzentrifugation vorgenommen werden. Die Fraktionen werden räumlich voneinander getrennt, z. B. durch Abpipettieren. Abschließend wird die Konzentration an A-Beta in der jeweiligen Fraktion durch eine vollständige Denaturierung der A-Beta-Spezies während einer im Anschluss an die Fraktionierung durchgeführten Umkehrphasen (RP-) HPLC bestimmt. Die Denaturierung der Aggregate kann z. B. mit 30 % Acetonitril und 0,1 % Trifluoressigsäure bei einer Säulentemperatur von 80 °C vollständig erfolgen und auf einer C8-Säule nach Hydrophobizität aufgetrennt werden. Eluierendes A-Beta wird mittels der UV-Absorption bei 215 nm detektiert. Die Peakflächenintegration kann mittels Agilent Chemstation Software erfolgen. Die Verrechnung daraus entstandener Werte mit der zuvor durchgeführten Kalibrierung erlaubt die Berechnung der Konzentration des in der jeweiligen Fraktion vorhandenen A-Beta. Je Fraktion sollte der Mittelwert aus mehreren z. B. sechs voneinander unabhängig durchgeführten Experimenten mit der sich ergebenen Standardabweichung berechnet werden. Der Vorteil der HPLC-Analyse liegt darin, dass man unabhängig vom Aggregationszustand und eines Lösungsmittels sehr sensitiv detektieren (z. B. ca. 20 nM oder 1,8 ng Aß1-42) und zuverlässig quantifizieren kann. Ein entscheidender Vorteil des Verfahrens besteht in der Kopplung von Dichtegradientenzentrifugation und Umkehrphasen HPLC, welche eine zuverlässige Quantifizierung auch von Aß-Oligomeren möglich macht.

Ergebnisse sind in der Tabelle 1 zusammengefasst. Sie zeigen, dass die Substanzen besonders effizient Oligomere eliminieren.

**Tabelle 1 (* = nicht erfindungsgemäß):**

| | Substanz / Peptid | QIAD: Reduktion von Oligomeren in % |
|---|---|---|
| 1 | D3 amidiert* | 56 |
| 2 | RD2 amidiert* | 78 |
| 3 | D3D3 amidiert | 98 |
| 4 | RD2RD2 amidiert* | 97 |
| 5 | RD2D3 amidiert | 100 |
| 6 | D3RD2 amidiert | 88 |

Eingesetzt wurden die Substanzen jeweils in der Konzentration von 32 µg/ml.

Die Ergebnisse zeigen zudem, dass die getesteten erfindungsgemäßen Dimere einen synergistischen Effekt im Vergleich zu den eingesetzten Monomeren bei gleicher Dosierung haben. Im Weiteren werden einige *in vivo* Daten zu den eingesetzten Peptiden vorgestellt, die die Wirksamkeit der erfindungsgemäßen Polymere, insbesondere die der Dimere, insbesondere die der Dimere gemäß der SEQ ID NO:4-8 belegen.

In Tierversuchen an verschiedenen transgenen Mausmodellen (Tabelle 2) wurde gezeigt, dass alle bisher getesteten amidierten Peptide in vivo aktiv sind:

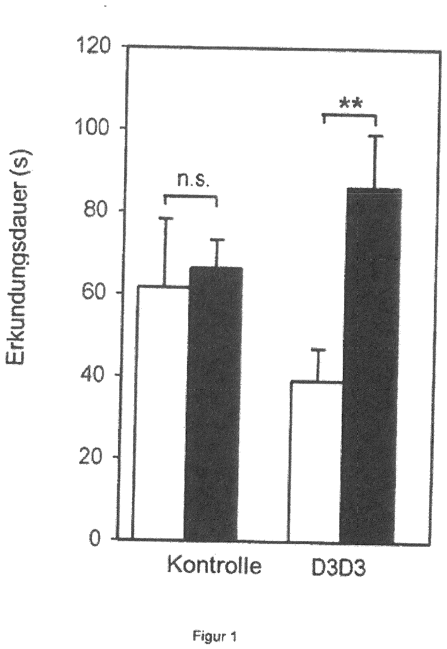

Figur 1: Hier ist das Ergebnis des sogenannten "object recognition tests" (ORT) zu sehen. Dabei wurden mit D3D3 (amidiert) behandelte Tiere (Details in Tabelle 2) und mit Placebo behandelte Tiere einzeln und getrennt voneinander in eine Box gesetzt, in der sich zwei Gegenstände befanden. Nachdem die Tiere Zeit hatten, beide Gegenstände zu erkunden, wurden die Tiere wieder aus der Box genommen und erst 24 Stunden später wieder eingesetzt. Davor wurde jedoch ein Objekt durch ein neues davon verschiedenes ausgetauscht. Tiere mit Gedächtnis erkunden nun bevorzugt das neue Objekt. Tiere ohne Gedächntnis an den Aufenthalt in der Box 24 Stunden zuvor verbringen gleich viel Zeit mit dem Erkunden des alten Objektes und des neuen Objektes. Gemessen wird die Zeit in Sekunden, die das Tier mit dem alten Objekt (weiße Balken) und dem neuen Objekt (schwarze Balken) verbringt. Es ist einddeutig zu erkennen, dass die Placebo-behandelten

Tiere gleich viel Zeit mit dem neuen und dem alten Objekt verbringen. Die D3D3-behandelten Tiere verbringen jedoch viel mehr Zeit mit dem neuen Objekt. Nur die D3D3-behandelten Tiere zeigten also ein funktionierendes Gedächtnis in dem Test.

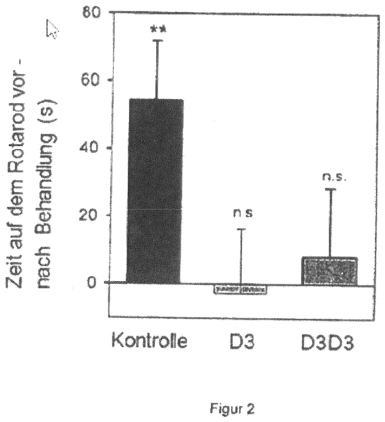

Figur 2: Im sogenannten "Rotarod"-Assay wird gemessen, wie lange ein Tier in der Lage ist, auf einem sich drehenden Stab zu balancieren, bevor sie herunter fallen. Damit kann man die neuromotorische Leistung des Tiers messen. Je länger das Tier balancieren kann, desto weniger stark sind seine Motoneuronen vom Phänotyp einer Neurodegeneration betroffen. Die Messung wurde vor und nach der Behandlung (Details zur Behandlung in Tabelle 2) durchgeführt. Die Differenz zwischen vor und nach der Behandlung ist in Sekunden aufgetragen. Der positive Wert für die Kontrollgruppe (schwarzer Balken) zeigt an, dass die unbehandelten Tiere vor der Behandlung weniger Degeneration zeigten als nach der Placebo-Behandlung. Die Degeneration ist also wie zu erwarten war, fortgeschritten. Die mit D3 (amidiert) und D3D3 (amidiert) behandelten Tiere zeigten jedoch keine Verschlechterung der neuromotorischen Symptome. Die Behandlung führte also in beiden Fällen zu einer Verlangsamung oder sogar zu einem Stopp der Neurodegeration.

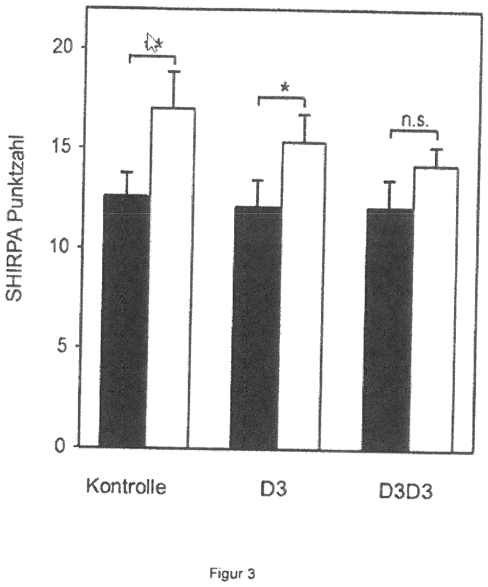

Figur 3 : Der SHIRPA-Test ist ein Test, in dem eine Reihe von Eigenschaften, unter anderem von Reflexen gestestet werden. Jede Eigenschaft wird getrennt von den anderen bewertet und jeweils Punkte vergeben. Je mehr Punkte, desto weiter fortgeschritten ist der Phänotyp, also die Neuro-Schädigung. Der SHIRPA-Test wurde vor (schwarze Balken) und nach (wei-ße Balken) der Behandlung (Details zur Behandlung in Tabelle 2) gemessen. Man kann erkennen, dass die mit D3D3 (amidiert) behandelten Tiere keine signifikante Verschlechterung des SHIRPA-Wertes zeigten. Die mit D3 (amidiert) und mit Placeobo behandelten Tiere zeigten allerdings eine mehr oder weniger starke Verschlechterung des SHIRPA-Wertes. D3D3 wirkt also effizienter als D3.

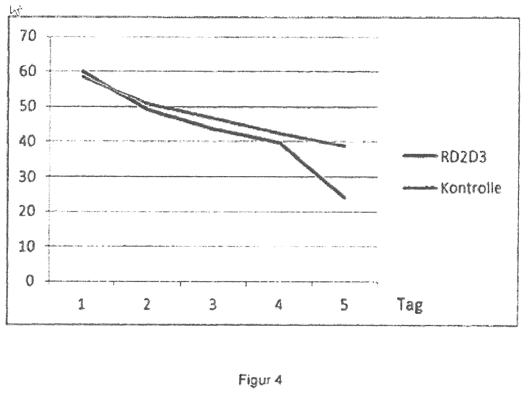

Figur 4: Mit dem sogannten "Morris water maze" wird das räumliche Gedächtnis von Tieren gemessen. Dabei wird die Zeit in Sekunden gemessen, die das Tier benötigt, eine dicht unter der Wasserobfläche versteckte Plattform in einem Wasserbottich wieder zu finden. Dies wird in mehreren Versuchen pro Tag an mehreren aufeinanderfolgenden Tagen gemessen. Eine statistische Auswertung ergibt dann, ob die behandelten Tiere im Vergleich zur Kontrollgruppe besser gelernt haben, die Plattform wiederzufinden. Aufgetragen ist die Suchzeit in Sekunden an fünf aufeinanderfolgenden Tagen. Die mit RD2D3 (amidiert) behandelten Tiere haben signifikant gelernt.

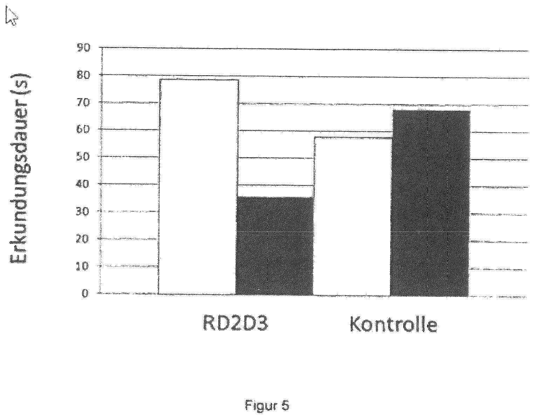

Figur 5: Hier ist das Ergebnis des sogenannten "object recognition tests" (ORT) zu sehen. Dabei wurden mit RD2D3 (amidiert) behandelte Tiere (Details in Tabelle 2) und mit Placebo behandelte Tiere einzeln und getrennt voneinander in eine Box gesetzt, in der sich zwei Gegenstände befanden. Nachdem die Tiere Zeit hatten, beide Gegenstände zu erkunden, wurden die Tiere wieder aus der Box genommen und erst 24 Stunden später wieder eingesetzt. Davor wurde jedoch ein Objekt durch ein neues davon verschiedenes ausgetauscht. Tiere mit Gedächtnis erkunden nun bevorzugt das neue Objekt. Tiere ohne Gedächtnis an den Aufenthalt in der Box 24 Stunden zuvor verbringen gleich viel Zeit mit dem Erkunden des alten Objektes und des neuen Objektes. Gemessen wird die Zeit in Sekunden, die das Tier mit dem alten Objekt (schwarze Balken) und dem neuen Objekt (weiße Balken) verbringt. Es ist eindeutig zu erkennen, dass die Placebo-behandeltenTiere gleich viel Zeit mit dem neuen und dem alten Objekt verbringen. Die RD2D3-behandelten Tiere verbringen jedoch viel mehr Zeit mit dem neuen Objekt. Nur die RD2D3-behandelten Tiere zeigten also ein funktionierendes Gedächtnis in dem Test.

Die amidierten Formen des D3 und des D3D3 sind in der Lage, in TBA-2.1-Mäusen während der Behandlungszeit von 4 Wochen das Fortschreiten des Phänotyps zu stoppen (siehe auch Figur 2).

Insbesondere konnte bestätigt werden, dass das Doppelpeptid D3D3 (amidiert) effizienter wirkt, als das einfache Peptid D3 (amidiert) und zwar im SHIRPA-Assay, ebenfalls im TBA-2.1-Tiermodell (Fig. 3). D3D3 konnte im SweDl-Mausmodell zeigen, dass es die Kognition (im "Object recognition test", Fig. 1) deutlich verbessert im Vergleich zu nicht-behandelten Mäusen. RD2D3 (amidiert) ist ebenfalls in der Lage, die Kognition von SweDl-Mäusen zu verbessern, was sowohl im Morris water maze (Fig. 4) als auch im Object recognition test (Fig. 5) gezeigt wurde.

### Weitere Ausführungsbeispiele:

Die nachfolgenden Schritte beziehen sich sowohl auf Affinitätsstudien als auch auf Studien zum Abbau besonders toxischer Amyloid-Beta-Oligomere.

Herstellung von Aß-Monomeren, Oligomeren und Fibrillen
1 mg lyophilisiertes Aß1-42 und N-terminal biotinyliertes Aß1-42 wurden jeweils in 1 ml 100% Hexafluorisopropanol (HFIP) gelöst und bei Raumtemperatur über Nacht gelöst. Für die Oligomer- und Fibrillenpräparation wurden nicht-biotinyliertes Aß mit N-terminal biotinyliertem Aß in einem Verhältnis von 1:10 verwendet und das HFIP abgedampft (Concentrator 5301 von Eppendorf). Der entstandene Aß-Film wurde in einer finalen Konzentration von 80 µM in Natriumphosphatpuffer (10 mM, pH 7,4) aufgenommen und inkubiert (RT, 600 rpm). Die Inkubationszeit betrug bei der Oligomerpräparation 3 h und bei der Fibrillenpräparation 24 h. Zur Präparation von Monomeren wurde 100% N-terminal biotinyliertes Aß1-42 ohne Inkubation verwendet.

### Dichtegradientenzentrifugation

Die Dichtegradientenzentrifugation wurde im Anschluss an die Aß Präparation durchgeführt, um die jeweiligen Aß Spezies ihrer Größe entsprechend aufzureinigen. Dazu wurde ein lodixanol-Gradient in 10 mM Natriumphosphatpuffer, pH 7.4, mit ansteigenden Konzentrationen von 50% bis 5% v/v lodixanol verwendet. 100 µl der Aß-Probe wurden aufgetragen und mittels Ultrazentrifugation aufgetrennt (3 h, 4 °C, 259.000 g). Im Anschluss wurde der Gradient in 14 Fraktionen zu je 140 µl fraktioniert. Monomere Aß befinden sich in den ersten beiden oberen Fraktionen, Aß-Oligomere in den Fraktionen 4 bis 6 und Aß-Fibrillen in den Fraktionen 11 bis 13.

### Immobilisierung für SPR-Spektroskopie (Oberflächenplasmonresonanz)

Für SPR-Spektroskopie wurde ein T200 von Biacore (GE Healthcare) verwendet. Die mittels Dichtegradientenzentrifugation gereinigten Aß-Spezies wurden direkt mittels Biotin-Streptavidin-Kopplung auf einen Sensor Chip (Series S Sensor Chips SA) nach Herstellerangaben immobilisiert. Als Laufpuffer diente 1X PBS. Die Beladung erfolgte bei 25 °C und einer Flussgeschwindigkeit von 5 µl/min. Im Anschluss wurden die Flusszellen über Nacht bei einem stetigen Fluss von 30 µl/min von unspezifisch gebundenem Liganden befreit.

### Bindungskinetiken

Bindungskinetiken wurden ebenfalls mittels SPR-Spektroskopie an einem T200 Gerät von Biacore (GE Healthcare) gemessen. Die Standardbedingungen sind 25 °C und eine Flussgeschwindigkeit von 30 µl/min. Verschiedene Lyophilisate der D-Peptide wurden im Laufpuffer 1X PBS aufgenommen und seriell verdünnt. Als Methode diente eine "Single-Cycle"-Kinetik, wobei fünf ansteigende Analyt-Konzentrationen über die immobilisierten Flusszellen gepumpt werden. Die Kontaktzeiten wurden je nach Analyt 90-120 s für Assoziation und Dissoziation und 1800-5400 s für die finale Dissoziation gewählt. Die Sensorgramme wurden doppelt referenziert mit Hilfe einer nicht beladenen Flusszelle und dem verwendeten Laufpuffer. Die Auswertung der Bindungskurven erfolgte mittels kinetischen Fit-Modellen (Heterogenes Bindemodell) unter Verwendung der Biacore T200 Evaluation Software (Version 2.0).

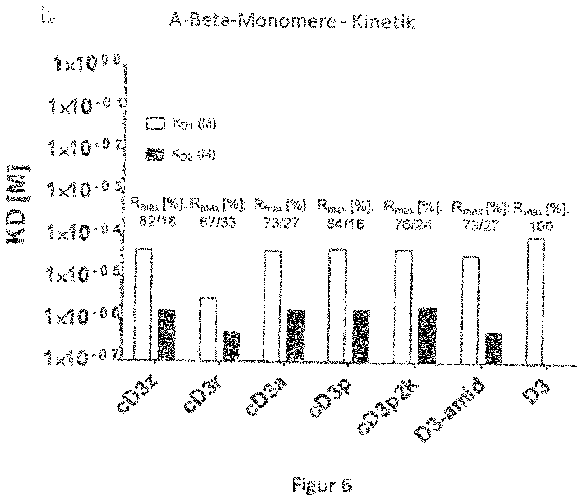

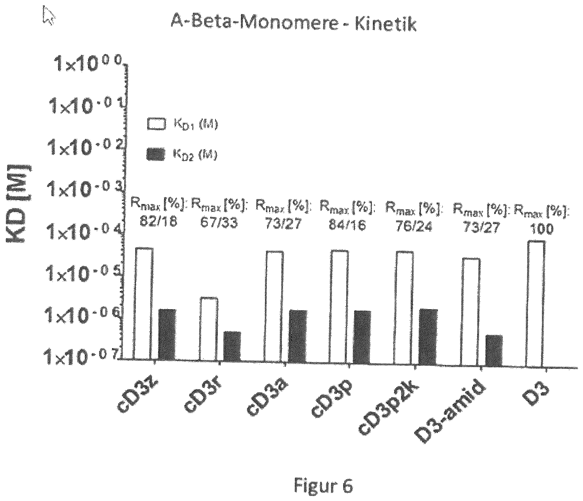

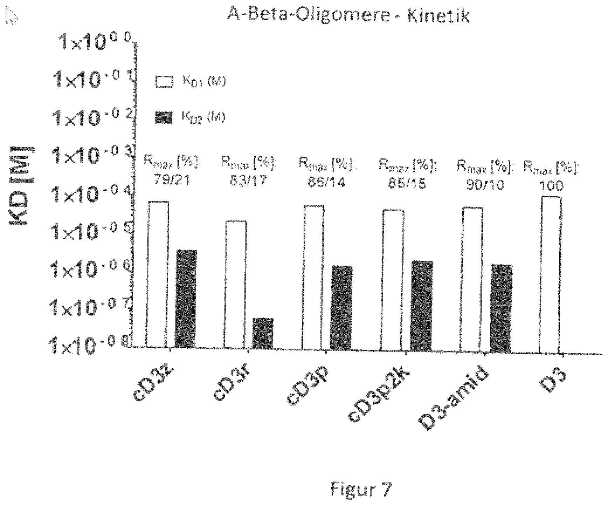

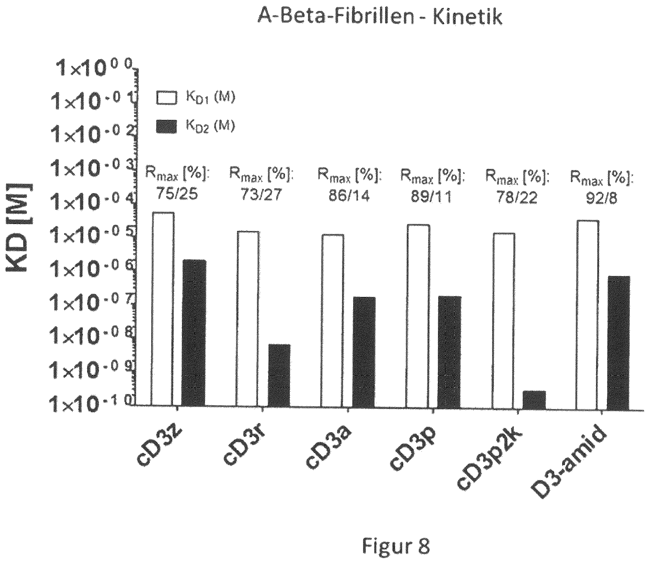

Die Figuren 6-8 (nicht erfindungsgemäß) zeigen die Ergebnisse zum Bindungsverhalten der Peptide (Affinitätsstudie). In den Figuren sind Daten zur kinetischen Auswertung der Bindungsstärke der verschiedenen Kandidaten an Amyloid-Beta-Monomere (Figur 6), Amyloid-Beta-Oligomere (Figur 7) und Amyloid-Beta-Fibrillen (Figur 8). Gezeigt sind jeweils die zwei Bindekonstanten, die beim Fitten eines heterogenen Bindemodells entstehen, und jeweils als weiße Balken und schwarze Balken aufgetragen sind. Wichtig ist, dass hier eine logarithmische Skala zu sehen ist, das bedeutet kleine Unterschiede in der Balkengröße sind große Unterschiede in der Dissoziationskonstanten K_{D}. Weiße Balken sind jeweils die low-afinitysites, also die niedrigaffinen Bindestellen und deren Bindungsstärke und die schwarzen Balken zeigen die hochaffinen Bindestellen und deren Bindungsstärke.

Es wird gezeigt, dass nur im Falle des linearen Peptids D3, bei dem das freie C-terminus eine negative Ladung aufweist, ein homogenes 1:1-Bindemodell ausreicht, um die Bindungskurven zu fitten.

Es wird außerdem gezeigt, dass in fast allen Fällen bei den verwendeten zyklisierten Peptiden die weißen Balken zu den niedrigaffinen Bindestellen ähnlich hoch sind. Die großen Unterschiede entstehen vielmehr bei den hochaffinen Bindestellen, die als schwarze Balken dargestellt sind.

Ferner wird deutlich, dass bei den Monomeren das zyklische cD3r (SEQ ID NO: 9) sehr gut abschneidet (Figur 6), also eine besonders hohe Affinität besitzt.

Bei den Oligomeren, die besonders interessant sind, bindet cD3r sogar um zwei Größenordnungen stärker als die anderen zyklisierten Peptide (Figur 7).

Auch bei den Fibrillen schneidet das cD3r sehr gut im Vergleich zu den anderen Peptiden ab, wobei hier als Besonderheit das Peptid cD3(P2K) (SEQ ID NO: 12) extrem gut abschneidet und bis in den sub-pM Bindebereich vorstößt (Figur 8).

Es wird außerdem gezeigt, dass die amidierte Form des D3 im Vergleich zur nicht amidierten Form des D3 an Amyloid-Beta-Monomer und Amyloid-Beta-Oligomer stärker bindet. Dies zeigt sich in dem niedrigeren K_{D1}-Wert für die low-affinity-site sowie der Existenz einer high-affinity-site, der eine K_{D2} im sub-µM Bereich hat.

Die Rₘₐₓ-Werte in den Figuren 6-8 geben an, wie stark der Beitrag des jeweiligen K_{D} zur Gesamtbeladungskapazität ist. Im Beispiel zu den Oligomeren (Figur 7) ist für das Peptid cD3z ein Rₘₐₓ von 79/21% angegeben. cD3z steht für das cD3 zero, das heißt zyklisiertes D3 ohne weitere Aminosäureanhänge. Der weiße Balken zeigt an, dass die low affinity site insgesamt 79% der RU Gesamtbeladungsstärke ergeben kann und K_{D2} insgesamt 21% der RU. Das bedeutet, dass für dieses Peptid etwa ein Verhältnis von 1:4 an Bindestellen vorliegt, das heißt es gibt etwa 4 mal so viele low-affinitiy-sites wie high-affinity-sites.

Derartige Unterscheidungen zwischen der low affinity site und der high affinity site treten bei den Peptiden auf.

Die Tatsache, dass sich beim Fitten der experimentellen Bindungsdaten für das lineare Peptid D3 keine high affinity site ergibt, kann damit erklärt werden, dass es entweder nicht an die high affinity site bindet, oder aber dass die Affinitäten zu high affinity site und low affinity site nicht unterscheidbar sind.

Aus diesen Daten wird deutlich, dass das lineare D3 bei Amyloid-Beta-Oligomer nur eine low affinity site bindet, nicht aber eine high affinity site.

**Tabelle 2 (* = nicht erfindungsgemäß)**

| Experiment Nummer | Peptid | Mausmodell | Dosis | watermaze | Object recognition | Plaque Pathologie | SHIRPA | Rotarod |
|---|---|---|---|---|---|---|---|---|
| 1* | D3 amidiert* | TBA | 0,1 mg/Tag | Nicht durchgeführt | Nicht durchgeführt | Nicht durchgeführt | | signifikant (Fig.2) |
| 2 | D3D3 amidiert | TgSwDI | 1 mg/4 Wochen | signifikant Tag 5 | signifikant (Fig.1) | Significant | Nicht durchgeführt | Nicht durchgeführt |
| 3 | D3D3 amidiert | TBA | 0,1 mg/Tag | Nicht durchgeführt | Nicht durchgeführt | Nicht durchgeführt | signifikant (Fiq.3) | signifikant (Fig.2) |
| 4 | RD2D3 amidiert | TgSwDI | 7 mg/4 Wochen | signifikant Tag 5 (Fig.4) | signifikant (Fig.5) | | Nicht durchgeführt | Nicht durchgeführt |

| | | | | | | | | |
|---|---|---|---|---|---|---|---|---|
| Die Substanzen wurden jeweils für 4 Wochen i.p. (Mikropumpe) verabreicht. | | | | | | | | |

## Patentansprüche

1. Peptid, enthaltend mindestens eine Aminosäuresequenz, welche an Amyloid-Beta-Spezies bindet und bei dem die negative Ladung der gewöhnlich am freien C-Terminus vorhandenen Carboxylgruppe durch Modifizierung entfernt wurde, so dass dort keine oder eine positive Ladung vorkommt, wobei am freien C-terminus an Stelle der Carboxylgruppe eine Säureamidgruppe (CONH2-Gruppe) vorliegt, enthaltend mindestens ein Monomer mit einer Aminosäuresequenz, ausgewählt aus der Gruppe bestehend aus SEQ ID NO: 4, SEQ ID NO: 5 und SEQ ID NO: 6.

2. Peptid nach Anspruch 1, **gekennzeichnet dadurch, dass** es aus D-enantiomeren Aminosäuren besteht.

3. Peptid nach einem der vorhergehenden Ansprüche, **dadurch gekennzeichnet, dass** dieses mit einer weiteren Substanz verknüpft ist.

4. Peptid nach einem der vorhergehenden Ansprüche, im Folgenden genannt "Polymer", **gekennzeichnet dadurch, dass** es mindestens zwei Monomere mit einer Aminosäuresequenz, ausgewählt aus der Gruppe bestehend aus SEQ ID NO: 4, SEQ ID NO: 5 und SEQ ID NO: 6 enthält, die an Amyloid-Beta-Spezies binden.

5. Peptid nach dem vorherigen Anspruch, **gekennzeichnet durch** ein Dimer aus zwei Monomeren mit einer Aminosäuresequenz, ausgewählt aus der Gruppe bestehend aus SEQ ID NO: 4, SEQ ID NO: 5 und SEQ ID NO: 6.

6. Peptid nach einem der Ansprüche 1 bis 3, **gekennzeichnet durch** ein Dimer aus zwei Monomeren, wobei ein Monomer ausgewählt ist aus der SEQ ID NO: 4, SEQ ID NO: 5 oder SEQ ID NO: 6 und ein Monomer ausgewählt ist aus SEQ ID NO: 7 oder SEQ ID NO: 8.

7. Peptid nach einem der vorangehenden Ansprüche zur Verwendung in der Medizin.

8. Peptid nach einem der vorangehenden Ansprüche zur Behandlung der Morbus Alzheimer.

9. Peptid nach einem der vorhergehenden Ansprüche, zur Verwendung in der Hemmung der Fibrillenbildung von Amyloid-Beta-Peptiden.

10. Peptid nach einem der vorangehenden Ansprüche zur Verwendung zur Bindung an aggregierte Amyloid-Beta-Peptide.

11. Verfahren zur Herstellung eines Peptids nach einem der vorherigen Ansprüche, **dadurch gekennzeichnet, dass** das Peptid durch Peptid-Synthese hergestellt wird.

12. Kit, enthaltend ein Peptid nach einem der Ansprüche 1 - 10.

13. Zusammensetzung, enthaltend ein Peptid nach einem der Ansprüche 1-10.

14. Peptid nach einem der Ansprüche 1 - 10 zur Verwendung als Sonde zur Identifizierung, quantitativen und/oder qualitativen Bestimmung von Amyloid-Beta-Fibrillen, Amyloid-Beta-Protofibrillen und/oder Amyloid-Beta-Oligomeren.

15. Peptid nach einem der Ansprüche 1- 10 zur Verwendung in der Verhinderung der Bildung von Amyloid-Beta-Fibrillen, -Protofibrillen, -Oligomeren und/oder Amyloid-Beta-Peptidaggregaten.

16. Peptid nach einem der Ansprüche 1 - 10 zur Verwendung in der Eliminierung und/oder Enttoxifizierung von toxischen Amyloid-Beta-Fibrillen, Protofibrillen, - Oligomeren und/oder Aggregaten.

17. Peptid zur Verwendung nach Anspruch 16 **dadurch gekennzeichnet, dass** Amyloid-Beta-Oligomere und/oder Aggregate mit dem Peptid nach einem der Ansprüche 1 - 10 amorphe, nicht toxische Aggregate bilden.

18. Peptid nach einem der Ansprüche 1 - 10 zur Verwendung als Therapeutikum und zur Prävention von Morbus Alzheimer.

## Claims

1. Peptide comprising at least one amino acid sequence which binds to amyloid beta species and in which the negative charge of the carboxyl group normally present at the free C-terminus has been removed by modification so that there is no charge or a positive charge, wherein an acid amide group (CONH2 group) is present at the free C-terminus in place of the carboxyl group,
comprising at least one monomer having an amino acid sequence selected from the group consisting of SEQ ID NO: 4, SEQ ID NO: 5 and SEQ ID NO: 6.

2. Peptide according to claim 1, **characterized in that** it consists of D-enantiomeric amino acids.

3. Peptide according to any one of the preceding claims, **characterized in that** it is linked to a further substance.

4. Peptide according to any one of the preceding claims, hereinafter referred to as "polymer", **characterized in that** it comprises at least two monomers having an amino acid sequence selected from the group consisting of SEQ ID NO: 4, SEQ ID NO: 5 and SEQ ID NO: 6 which bind to amyloid beta species.

5. Peptide according to the preceding claim, **characterized by** a dimer of two monomers having an amino acid sequence selected from the group consisting of SEQ ID NO: 4, SEQ ID NO: 5 and SEQ ID NO: 6.

6. Peptide according to any one of claims 1 to 3, **characterized by** a dimer of two monomers, wherein one monomer is selected from SEQ ID NO: 4, SEQ ID NO: 5 or SEQ ID NO: 6 and one monomer is selected from SEQ ID NO: 7 or SEQ ID NO: 8.

7. Peptide according to any one of the preceding claims for use in medicine.

8. Peptide according to any one of the preceding claims for the treatment of Alzheimer's disease.

9. Peptide according to any one of the preceding claims, for use in inhibiting fibril formation of amyloid beta peptides.

10. Peptide according to any one of the preceding claims, for use in binding to aggregated amyloid beta peptides.

11. Method for the preparation of a peptide according to any one of the preceding claims, **characterized in that** the peptide is prepared by peptide synthesis.

12. Kit comprising a peptide according to any one of claims 1 - 10.

13. Composition comprising a peptide according to any one of claims 1-10.

14. Peptide according to any one of claims 1 - 10 for use as a probe for the identification, quantitative and/or qualitative determination of amyloid beta fibrils, amyloid beta protofibrils and/or amyloid beta oligomers.

15. Peptide according to any one of claims 1 - 10 for use in the prevention of the formation of amyloid beta fibrils, protofibrils, oligomers and/or amyloid beta peptide aggregates.

16. Peptide according to any one of claims 1 - 10 for use in the elimination and/or detoxification of toxic amyloid beta fibrils, protofibrils, -oligomers and/or aggregates.

17. Peptide for use according to claim 16 **characterized in that** amyloid beta oligomers and/or aggregates form amorphous non-toxic aggregates with the peptide according to any one of claims 1 - 10.

18. Peptide according to any one of claims 1 - 10 for use as a therapeutic agent and for the prevention of Alzheimer's disease.

## Revendications

1. Peptide contenant au moins une séquence d'acides aminés qui se lie aux espèces amyloïdes bêta et dans lequel la charge négative du groupe carboxyle habituellement présent au terminus C libre a été éliminée par modification, de sorte qu'aucune charge ou bien une charge positive y est présente,
un groupe amide d'acide (groupe CONH2) étant présent au terminus C libre à la place du groupe carboxyle,
contenant au moins un monomère ayant une séquence d'acides aminés choisie dans le groupe constitué par SEQ ID NO : 4, SEQ ID NO : 5 et SEQ ID NO : 6.

2. Peptide selon la revendication 1,
**caractérisé en ce qu'**il est constitué d'acides aminés D-énantiomères.

3. Peptide selon l'une des revendications précédentes,
**caractérisé en ce qu'**il est lié à une autre substance.

4. Peptide selon l'une des revendications précédentes, ci-après dénommé "polymère",
**caractérisé en ce qu'**il contient au moins deux monomères ayant une séquence d'acides aminés, choisie dans le groupe constitué par SEQ ID NO : 4, SEQ ID NO : 5 et SEQ ID NO : 6, qui se lient aux espèces amyloïdes bêta.

5. Peptide selon la revendication précédente,
**caractérisé par** un dimère de deux monomères ayant une séquence d'acides aminés, choisie dans le groupe constitué par SEQ ID NO : 4, SEQ ID NO : 5 et SEQ ID NO : 6.

6. Peptide selon l'une des revendications 1 à 3,
**caractérisé par** un dimère de deux monomères, un monomère étant choisi parmi SEQ ID NO : 4, SEQ ID NO : 5 ou SEQ ID NO : 6, et un monomère étant choisi parmi SEQ ID NO : 7 ou SEQ ID NO : 8.

7. Peptide selon l'une des revendications précédentes, destiné à être utilisé en médecine.

8. Peptide selon l'une des revendications précédentes pour le traitement de la maladie d'Alzheimer.

9. Peptide selon l'une des revendications précédentes, destiné à être utilisé pour inhiber la formation de fibrilles de peptides amyloïdes bêta.

10. Peptide selon l'une des revendications précédentes, destiné à se lier à des peptides amyloïdes bêta agrégés.

11. Procédé de préparation d'un peptide selon l'une des revendications précédentes,
**caractérisé en ce que** le peptide est préparé par synthèse peptidique.

12. Kit contenant un peptide selon l'une des revendications 1 à 10.

13. Composition contenant un peptide selon l'une des revendications 1 à 10.

14. Peptide selon l'une des revendications 1 à 10, destiné à être utilisé comme sonde pour l'identification, la détermination quantitative et/ou qualitative de fibrilles amyloïdes bêta, de protofibrilles amyloïdes bêta et/ou d'oligomères amyloïdes bêta.

15. Peptide selon l'une des revendications 1 à 10, destiné à être utilisé pour empêcher la formation de fibrilles, de protofibrilles, d'oligomères amyloïdes bêta et/ou d'agrégats de peptides amyloïdes bêta.

16. Peptide selon l'une des revendications 1 à 10, destiné à être utilisé pour l'élimination et/ou la détoxification de fibrilles, de protofibrilles, d'oligomères et/ou d'agrégats amyloïdes bêta toxiques.

17. Peptide pour son utilisation selon la revendication 16,
**caractérisé en ce que** les oligomères et/ou agrégats amyloïdes bêta forment, avec le peptide selon l'une des revendications 1 à 10, des agrégats amorphes non toxiques.

18. Peptide selon l'une des revendications 1 à 10, destiné à être utilisé comme agent thérapeutique et pour la prévention de la maladie d'Alzheimer.
